# EUROPEAN PATENT SPECIFICATION

(11) **EP 2 319 331 B1**
(45) Date of publication and mention of the grant of the patent: **14.08.2013**
(21) Application number: 09175548.8
(22) Date of filing: 10.11.2009
(51) Int. Cl.: A23L 1/29, A23L 1/30, A23L 1/305

(54) **Ready-to-use therapeutic food (RUTF) product for treating severe acute malnutrition in children aged 6-12 months and 1-3 years**
Benutzungsfertiges therapeutisches Lebensmittelprodukt zur Behandlung einer schweren Fehlernährung bei Kindern zwischen 6 und 12 Monaten und 1 bis 3 Jahren
Produit d'aliment thérapeutique prêt à être utilisé pour traiter la malnutrition aiguë sévère chez les enfants âgés de 6 à 12 mois et de 1 à 3 ans

(43) Date of publication of application: 11.05.2011
(73) Proprietor: Al Ketbi, Sana'a, Abu Dhabi (AE)
(72) Inventor: Al Ketbi, Sana'a, Abu Dhabi (AE)
(74) Representative: Dupuis-Latour, Dominique

(56) References cited:
- WO-A-03/079818
- WO-A-2006/014878
- WO-A-2006/054105
- WO-A-2007/103688
- WO-A-2008/076579
- FR-A- 2 644 983

## Description

### BACKGROUND OF THE INVENTION

The present invention generally relates to recipes and food technologies for producing supplementary food to treat undernourished children.

### Field of the invention

Today, malnutrition is recognized as a major global public health issue. Malnutrition is a direct cause of an important proportion of mortality among children less than five years of age.

In a Joint Statement of June 2007 on community-based treatment and care of children with severe acute malnutrition by WHO, WFP, UNICEF and SCN, a strategy based on the use of RUTF (Ready-to-Use Therapeutic Food) was endorsed as the way forward to tackle Severe Acute Malnutrition (SAM).

A needs assessment in therapeutic food shows an exponential increase in the consumption of these products, which can create serious stresses in the supply chain; this was the case in the mid-year 2008, where production capacity was totally overstretched in the context of a humanitarian emergency in Ethiopia. It is becoming clearly urgent to increase and diversify the capacity to produce nutritional pastes, and agencies like NGOs, and above all UNICEF itself can no longer continue to depend on too limited sources of supply.

Further, the presently available RUTF products are rather expensive, which is obviously a major concern given their humanitarian aim.

### Background art

There presently exist a few commercially available forms of RUTF products, which all have a number of disadvantages.

One of these RUTF products, which comes in the form of a compressed powder, is manufactured by Compact A/S in Bergen, Norway and sold under trade name *BP100.* This product is well suited to the treatment of acute malnutrition, however the facts show that for being given to younger children the product is generally prepared as a porridge by diluting the powder in water prior to consumption. This has the disadvantage to make it subject to (i) potential bacterial contamination and (ii) errors of dosage that may conduct to hyperosmolarity of the product.

Another presently available commercial form of RUTF is the so-called *Plumpy'nut* (registered trademark) product, which is a peanut-based paste developed by Institut de Recherche et Développement in Paris, France and company Nutriset in Malaunay, France. This product, which is disclosed in EP 1 032 280 A1 (corresponding to US 6 346 284 B1), is mainly featured by a low osmolarity and comprises a mixture of food-grade products essentially including peanut paste, vegetable oil, powdered milk, powdered sugar, vitamins and minerals.

However, set apart the fact that this product has not always been highly-accepted, it is prone to contamination by aflatoxins, which complicates quality assurance. As a matter of fact, virtually all sources of commercial peanut butter contain minute quantities of aflatoxins, which are naturally occurring mycotoxins that could lead to carcinogenic and DNA-mutatory effects in case of chronic exposure to excessive levels. Also, younger children are particularly affected by exposure to aflatoxins, which may lead to stunted growth and delayed development.

There is also growing concern about possible allergic reaction to peanut and about their high phytate:zinc ratio, which increases the risks of binding all micronutrients thereby reducing their suitability.

WO 2006/054105 A1 and WO 2003/079818 A1 (Galactogen Products Ltd.) disclose various formulations of energy bars for enhancing endurance and resistance to fatigue.

However, even if these formulations basically contain a mixture of carbohydrates, proteins, lipids, etc. as in most nutritional compositions, they are not at all adapted for a therapeutic use against malnutrition in children, all the more in the very specific context of humanitarian emergencies.

In particular, this context requires products having: i) a high energy concentration, in order to minimize transport and storage costs, ii) a long shelf-life - at least several years - in order to enable long-term storage making the product available at any moment in case of humanitarian emergency, and iii) a "ready-to-use" form, i.e. they may be used as such by any person, without addition of water (in order to avoid bacterial contamination), in a pasty form widely acceptable (not too hard to chew, not too sticky, no need to drink when eating, etc.).

The compositions of WO 2006/054105 A1 and WO 2003/079818 A1 do not meet these general requirements (as e.g. opposed to the specific RUTF product disclosed in above-mentioned EP 1 032 280 A1).

### SUMMARY OF THE INVENTION

It is therefore an object of the invention to provide a new formulation that can be produced in a cost-effective way, locally and from local raw material, rich in protein, energy dense and suitable for treating acutely malnourished children from 6 months to 3 years of age, while overcoming the multiple drawbacks of the known products.

Essentially, after having evaluated and tested numerous food raw materials in order to achieve the product development and to reach nutritionally suitable mix for RUTF, the inventor found that a ready-to-use, pasty product having the four following basic ingredients would meet all such requirements:
- a major ingredient originating from dates and including less than 5% w/w crude fibers;
- a protein supplement representing at least half of the protein content of the composition;
- an energy supplement including lipids provided from vegetable oil, and carbohydrates; and
- vitamin and mineral supplements, in a quantity corresponding to Recommended Daily Allowance for children aged 6-12 months, or 1-3 years, respectively.

The major ingredient is date powder/paste mix obtained by steaming dates, then vacuum-drying and grinding the resulting product, said major ingredient originating from dates being in a quantity of 22-39% w/w.

Typically, the composition of the invention does not substantially includes added liquid ingredients other than the vegetable oil, the RUTF product being adapted for consumption without prior addition of liquid.

The RUTF product of the invention includes less than 20 ppb, preferentially less than 10 ppb, most preferentially less than 5 ppb, aflatoxins.

The protein supplement representing at least half of total protein content (i.e. UN 2008 specifications for ready-to-use food designed for rehabilitation of SAM cases) is preferably a supplement of animal origin including whey and/or skimmed powder milk, in particular 3-15% w/w whey and 5-20% w/w skimmed powder milk. Soya derivatives may also be provided an as alternative protein supplement.

The lipids for the energy supplement are preferably from sunflower oil in a quantity of 15-40% w/w.

The product may include 4-21% w/w sugar and/or maltodextrose as carbohydrates.

A typical w/w composition of the inventive product, totalling 100%, is: 22-39% of the major ingredient; 8-35% protein supplement; 19-61% energy supplement including lipids and carbohydrates; and 1-2% vitamin and mineral supplements.

A preferred composition is: 22-39% date powder/paste mix; 3-15% whey; 5-20% powder skimmed milk; 15-40% sunflower oil; 4-21% sugar and/or maltodextrose; and 1-2% vitamins, minerals, flavourings and enzyme mix. The most preferred composition is: 33% date powder/paste mix; 12% whey; 10% powder skimmed milk; 34% sunflower oil; 10% sugar and/or maltodextrose; and 1% vitamins, minerals, flavourings and enzyme mix.

### DETAILED DESCRIPTION OF

### A PREFERRED EMBODIMENT OF THE INVENTION

The foregoing and other objects, aspects and advantages of the invention will be better understood from the following detailed description of a preferred, however non-limiting, embodiment of the invention.

As mentioned above, the idea behind the invention is to develop a new and cost-effective formulation that can be produced locally and using local raw material, rich in protein, energy dense and suitable for treating acutely malnourished children from 6 months to 3 years of age.

The invented product has four basic ingredients:
- dates as a main ingredient;
- a protein supplement, typically from animal origin such as whey and skimmed milk, representing at least half of total protein content, according to UN 2008 specifications for ready-to-use food designed for rehabilitation of SAM cases (soya derivates can replace the milk and whey in case of allergy to these products);
- an energy supplement, typically including vegetable oil and sugar to increase the energy concentration of the mix; and
- vitamin and mineral supplements.

In the following, "sugar" is generally intended as plain sugar, i.e. sucrose (saccharose) obtained from sugar beet, sugar cane and other sources, but this word may also encompass other oses that have equivalent nutritional properties, in particular maltodextrose. Granulometry may correspond to icing sugar or regular sugar.

A preferred, yet non-limiting, quantitative formula has the following w/w composition (totalling 100%):
- 33% date powder/paste mix;
- 12% whey;
- 10% powder skimmed milk;
- 34% sunflower oil;
- 10% sugar and/or maltodextrose; and
- 1% vitamins, minerals, flavourings and enzyme mix.

The preferred process for manufacturing this RUTF product will be now described.

The first step is the preparation of the dates. After washing, dates are submitted to pressurized water steam and are pitted. This procedure reduces the content in fibers of the paste obtained.

The paste is then dried using vacuum. This technique not only dehydrates the dates, it also has the merit of preserving the nutritional quality and the taste of the paste product. Finally the dry product is ground and transformed in a soft powder/paste mix that will enter in the composition of the RUTF of the invention.

Separately, the mix of vitamins, minerals and enzymes is blended with warm oil, resulting in a fortified oil supplement, which is spread throughout the remaining ingredients of the inventive formula (skimmed powder milk, sugar and whey) to form a homogenous blend, with an optimal homogeneity and dispersion of vitamins and minerals in the resulting blend. Finally, the resulting mixture is added and remixed with a larger quantity of the previously prepared powder ingredients.

The product is then sterilized by means of heating before packaging.

For the final packaging step, a paste filling machine is used to fill the product into double-layer, airtight sachets which include an aluminum layer for protection against UV, light and humidity. This type of packaging will assure an optimal shelf life and preservation of the product.

A particular feature of the invention is the provision of two different formulations, each formulation being designed to respond to the nutritional needs of a specific age group:
- a first package adapted for children aged 6-12 months, with a sachet of 50 g unitary content and an adequate composition in terms of vitamins, minerals and enzymes in accordance with Recommended Daily Allowance (RDA) specific to this age group;
- a second package adapted to children aged 1-3 years, with a sachet of 100 g unitary content and an adequate composition in terms of vitamins, minerals and enzymes content according to RDA specific to the 1-3 years age group, which composition differs from the formulation designated for the of 6-12 month age group.

It should be noted that each package shell contains only the needed dose for a single consumption (meal) and not the total daily needed dose, as opposed to presently available RUTF products. Such choice ensures a correct consumed dose and offers better hygiene and conservation of the product, with much lower chances of bacterial contamination. Further, individual packaging makes the product not as easily shared (diverted) by other members of a family.

The difference between the two formulations in terms of (i) unitary dosage and (ii) vitamins, minerals and enzymes content is a feature specific to the present invention, which is omitted and neglected by the other commercially available RUTF products.

The RUTF product according to the teachings of the invention has multiple advantages over known, presently available RUTF products, namely:
- good nutritional quality (i.e. protein, energy density and micronutrient content), that responds to current specifications for the management of Severe Acute Malnutrition (energy density level of 520kcal/100g);
- balanced low/high molecular weight ratio of proteins used in the formulation;
- use of sunflower oil enables to meet (n-3) and (n-6) fatty acids requirement of the recommendation of the UN nutritional standards (Codex), namely "at least 3 to 10% of total energy should be provided by (n-6) fatty acids and 0.3 to 2.5% by (n-3) fatty acids";
- improved process parameters to increase starch gelatinization by various technologies (e.g. extrusion cooking);
- anti-nutritional factors level, mainly fibers and phytate levels, in line with the stated international specifications (Crude fiber: used 5 g/100 g maximum, according to CAC/GL 08-1991);
- aflatoxins under the level of 5 ppb;
- no known allergies;
- stable and microbiologically safe, due to low surface-to-volume ratio, reduced exposure to oxygen, fat coated, and total exclusion of water;
- long shelf life (24 months), even without refrigeration;
- optimal viscosity and consistency, with a texture suitable for feeding children (easy to swallow);
- highly palatable with a good taste, resulting in an optimal acceptability that permits high-level consumption and catch-up growth during rehabilitation;
- does not require any additional processing or preparation prior to feeding;
- the quantity distributed to each child is easy to calculate based on the weight: one simply needs to open the sachet by cutting one corner, and eat the paste;
- cost-effective, since it requires for its production simple food manufacturing facilities which are available in developing countries. In particular the RUTF product of the invention may be produced at a final cost up to 40% cheaper than the presently available RUTF products, which are typically in the expensive range of 3,000 euros per metric ton.

## Claims

1. A food product, for use as a Ready-to-Use Therapeutic Food (RUTF) in the therapy of Severe Acute Malnutrition (SAM) in children aged 6-12 months and 1-3 years, said food product being in the form of a ready-to-use, pasty composition,
**characterized by** the following w/w composition totalling 100%:
- 22-39% of a date powder/paste mix including less than 5% w/w crude fibers, obtained by steaming and pitting dates, then vacuum-drying and grinding the resulting product;
- 8-35% of a protein supplement accounting for at least half of the total protein content of the composition, said protein content being in compliance with specifications for RUTF designed for rehabilitation of SAM cases;
- 19-61% of an energy supplement including lipids provided from vegetable oil, and carbohydrates; and
- 1-2% vitamin and mineral supplements, in a quantity corresponding to Recommended Daily Allowance for children aged 6-12 months, or 1-3 years, respectively,
wherein said ready-to-use, pasty composition does not include added liquid ingredients other than said vegetable oil, and
said product is adapted for consumption without prior addition of liquid.

2. The food product of claim 1, including less than 20 ppb, preferentially less than 10 ppb, most preferentially less than 5 ppb, aflatoxins.

3. The food product of claim 1, wherein the protein supplement is a supplement of animal origin including whey and/or skimmed milk.

4. The food product of claim 3, including 3-15% w/w whey and 5-20% w/w skimmed powder milk.

5. The food product of claim 1, wherein the protein supplement includes soya derivatives.

6. The food product of claim 1, wherein said vegetable oil for the energy supplement includes sunflower oil.

7. The food product of claim 6, wherein said vegetable oil for the energy supplement includes 15-40% w/w sunflower oil.

8. The food product of claim 1, including 4-21% w/w sugar and/or maltodextrose as carbohydrates.

9. The food product of claim 1, having the following w/w composition totalling 100%:
- 22-39% date powder/paste mix;
- 3-15% whey;
- 5-20% powder skimmed milk;
- 15-40% sunflower oil;
- 4-21 % sugar and/or maltodextrose; and
- 1-2% vitamins, minerals, flavourings and enzyme mix.

10. The food product of claim 9, having the following w/w composition totalling 100%:
- 33% date powder/paste mix;
- 12% whey;
- 10% powder skimmed milk;
- 34% sunflower oil;
- 10% sugar and/or maltodextrose; and
- 1% vitamins, minerals, flavourings and enzyme mix.

## Patentansprüche

1. Lebensmittelprodukt zur Verwendung als gebrauchsfertiges therapeutisches Lebensmittelprodukt (Ready-to-Use Therapeutic Food (RUTF)) in der Therapie von schwerer Fehlernährung (Severe Acute Malnutrition (SAM)) bei Kindern im Alter von 6-12 Monaten und 1-3 Jahren, wobei das Lebensmittelprodukt in Form einer gebrauchsfertigen pastösen Zusammensetzung vorliegt, **dadurch gekennzeichnet, dass** es die folgende Zusammensetzung (w/w), die insgesamt 100% ausmacht, aufweist:
- 22-39% einer Dattelpulver/-pastenmischung, die weniger als 5% (w/w) Ballaststoffe aufweist und die durch Dämpfen und Entkernen von Datteln und anschließende Vakuumtrocknung und Vermahlung des erhaltenen Produkts erhalten wird;
- 8-35% eines Proteinzusatzes, der mindestens die Hälfte des Gesamtproteingehalts der Zusammensetzung ausmacht, wobei der Proteingehalt den Vorgaben für ein RUTF, das für die Rehabilitation von SAM-Fällen konzipiert ist, entspricht;
- 19-61% eines Energiezusatzes, der Lipide, die aus Pflanzenöl stammen, und Kohlenhydrate umfasst; und
- 1-2% Vitamin- und Mineralstoffzusätze in einer Menge, die dem empfohlenen Tagesbedarf für Kinder im Alter von 6-12 Monaten bzw. 1-3 Jahren entspricht,
wobei die gebrauchsfertige pastöse Zusammensetzung außer dem Pflanzenöl keine zugesetzten flüssigen Bestandteile umfasst, und
wobei das Produkt für den Konsum ohne zuvorige Flüssigkeitszugabe adaptiert ist.

2. Lebensmittelprodukt nach Anspruch 1, das weniger als 20 ppb, vorzugweise weniger als 10 ppb, am stärksten bevorzugt weniger als 5 ppb, Aflatoxine umfasst.

3. Lebensmittelprodukt nach Anspruch 1, wobei es sich bei dem Proteinzusatz um einen Zusatz tierischen Ursprungs, darunter Molke und/oder Magermilch, handelt.

4. Lebensmittelprodukt nach Anspruch 3, das 3-15% (w/w) Molke und 5-20% (w/w) Magermilchpulver umfasst.

5. Lebensmittelprodukt nach Anspruch 1, wobei der Proteinzusatz Sojaderivate umfasst.

6. Lebensmittelprodukt nach Anspruch 1, wobei das pflanzliche Öl für den Energiezusatz Sonnenblumenöl umfasst.

7. Lebensmittelprodukt nach Anspruch 6, wobei das pflanzliche Öl für den Energiezusatz 15-40% (w/w) Sonnenblumenöl umfasst.

8. Lebensmittelprodukt nach Anspruch 1, das 4-21% (w/w) Zucker und/oder Maltodextrose als Kohlenhydrate umfasst.

9. Lebensmittelprodukt nach Anspruch 1, das die folgende w/w-Zusammensetzung, die insgesamt 100% ausmacht, aufweist:
- 22-39% Dattelpulver/-pastenmischung;
- 3-15% Molke;
- 5-20% Magermilchpulver;
- 15-40% Sonnenblumenöl;
- 4-21% Zucker und/oder Maltodextrose; und
- 1-2% Vitamine, Mineralstoffe, Geschmacksstoffe und Enzymmischung.

10. Lebensmittelprodukt nach Anspruch 9, das die folgende w/w-Zusammensetzung, die insgesamt 100% ausmacht, aufweist:
- 33% Dattelpulver/-pastenmischung;
- 12% Molke;
- 10% Magermilchpulver;
- 34% Sonnenblumenöl;
- 10% Zucker und/oder Maltodextrose; und
- 1% Vitamine, Mineralstoffe, Geschmacksstoffe und Enzymmischung.

## Revendications

1. Produit alimentaire pour utilisation en tant qu'aliment thérapeutique prêt à l'emploi (ATPE) dans le traitement de la malnutrition aiguë sévère (MAS) chez les enfants âgés de 6 à 12 mois et de 1 à 3 ans, ledit produit alimentaire se présentant sous la forme d'une composition pâteuse prête à l'emploi, **caractérisé par** la composition suivante, en p/p, le total faisant 100 % :
- 22-39 % d'un mélange de poudre/de pâte de datte, comprenant moins de 5 % p/p de fibres brutes, obtenu par ébouillantage et dénoyautage de dattes, puis séchage sous vide et broyage du produit obtenu,
- 8-35 % d'un supplément protéique, comptant pour au moins la moitié de la teneur totale en protéines de la composition, ladite teneur en protéines étant conforme aux spécifications portant sur les ATPE conçus pour réhabilitation de cas de MAS,
- 19-61 % d'un supplément énergétique comprenant des lipides fournis par une huile végétale, et des hydrates de carbone, et
- 1-2 % de suppléments vitaminiques et minéraux, en une quantité correspondant à une dose quotidienne recommandée pour les enfants âgés respectivement de 6 à 12 mois ou de 1 à 3 ans,
où ladite composition pâteuse prête à l'emploi ne comprend pas d'ingrédients liquides ajoutés autres que ladite huile végétale, et
ledit produit étant apte à être consommé sans addition préalable de liquide.

2. Produit alimentaire selon la revendication 1, comprenant moins de 20 ppb, de préférence moins de 10 ppb, tout spécialement moins de 5 ppb d'aflatoxines.

3. Produit alimentaire selon la revendication 1, dans lequel le supplément protéique est un supplément d'origine animale comprenant du lactosérum et/ou du lait écrémé.

4. Produit alimentaire selon la revendication 3, comprenant 3-15 % p/p de lactosérum et 5-20 % p/p de lait écrémé en poudre.

5. Produit alimentaire selon la revendication 1, dans lequel le supplément protéique comprend des dérivés du soja.

6. Produit alimentaire selon la revendication 1, dans lequel ladite huile végétale pour le supplément énergétique comprend de l'huile de tournesol.

7. Produit alimentaire selon la revendication 6, dans lequel ladite huile végétale pour le supplément énergétique comprend 15-40 % p/p d'huile de tournesol.

8. Produit alimentaire selon la revendication 1, comprenant 4-21 % p/p de sucre et/ou de maltodextrose en tant qu'hydrates de carbone.

9. Produit alimentaire selon la revendication 1, ayant la composition suivante, en p/p, le total faisant 100 % :
- 22-39 % d'un mélange poudre/pâte de datte,
- 3-15 % de lactosérum,
- 5-20 % de lait écrémé en poudre,
- 15-40 % d'huile de tournesol,
- 4-21 % de sucre et/ou de maltodextrose, et
- 1-2 % de vitamines, de minéraux, d'arômes et d'un mélange enzymatique.

10. Produit alimentaire selon la revendication 9, ayant la composition suivante, en p/p, le total faisant 100 % :
- 33 % d'un mélange poudre/pâte de datte,
- 12 % de lactosérum,
- 10 % de lait écrémé en poudre,
- 34 % d'huile de tournesol,
- 10 % de sucre et/ou de maltodextrose, et
- 1 % de vitamines, de minéraux, d'arômes et d'un mélange enzymatique.
